# EUROPÄISCHE PATENTANMELDUNG

(11) **EP 1 068 862 A2**
(43) Veröffentlichungstag der Anmeldung: **17.01.2001**
(21) Anmeldenummer: 00109698.1
(22) Anmeldetag: 08.05.2000
(51) Int. Cl.: A61K 7/32

(54) **Desodorierende Wirkstoffkombination und deren Verwendung**

(30) Priorität: 28.05.1999 DE 19924496
(71) Anmelder: Wella Aktiengesellschaft, 64274 Darmstadt (DE)
(72) Erfinder: Diehl, Ilse, 64560 Riedstadt (DE); Bimczok, Rudolf, 64342 Seeheim-Jugenheim (DE)

(57) **Zusammenfassung**

Wirkstoffkombination als wirksames Prinzip zur Verwendung in kosmetischen Desodorantien auf der Basis eines synergistischen Gemisches aus (A) mindestens einem mehrwertigen Alkohol mit 5 bis 15 Kohlenstoffatomen, insbesondere einem 1,2-Diol, und (B) mindestens einem Citronensäuretrialkylester.

Beansprucht wird ferner ein Verfahren zur Bekämpfung des durch mikrobielle Zersetzung apokrinen Schweißes hervorgerufenen menschlichen Körpergeruches, dadurch gekennzeichnet, daß eine wirksame Menge des Gemisches aus (A) und (B), gegebenenfalls in einem geeigneten kosmetischen Träger, auf die Haut aufgetragen wird.

## Beschreibung

Die vorliegende Erfindung betrifft eine desodorierende Wirkstoffkombination, insbesondere eine Wirkstoffkombination als wirksames Prinzip zur Verwendung in kosmetischen Desodorantien.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten daher nur bei sehr starker Transpiration angewandt werden.

Durch die Verwendung antimikrobieller Stoffe in kosmetischen Desodorantien kann die Bakterienflora auf der Haut reduziert werden. Dabei sollten im Idealfall nur die geruchverursachenden Mikroorganismen wirksam reduziert werden. In der Praxis hat sich aber herausgestellt, daß die gesamte Mikroflora der Haut beeinträchtigt werden kann. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt eine bestimmtes Flair zu geben.

Die Parfümierung wirkstoffhaltiger kosmetischer Zubereitungen, insbesondere kosmetischer Desodorantien, ist allerdings nicht selten problematisch, weil Wirkstoffe und Parfümbestandteile gelegentlich miteinander reagieren und einander unwirksam machen können.

In der DE-OS 43 20 744 wird über die Verwendung von Alkandiolen mit 5 bis 10 C-Atomen wie 1,2-Pentandiol in kosmetischen Mittel wegen ihrer hautbefeuchtenden Wirkung berichtet.

Gute Desodorantien sollen folgende Bedingungen erfüllen:
1) Sie sollten eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw..

Aufgabe der vorliegenden Erfindung war es, kosmetische Desodorantien zur Verfügung zu stellen, welche die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war es, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspinerend wirkenden Formulierungen wesentlichen Parfümbestandteilen.

Noch eine weitere Aufgabe der Erfindung war es, kosmetische Desodorantien zur Verfügung zu stellen, welche über einen längeren Zeitraum, und zwar in der Größenordnung von mindestens 16 Stunden, vorzugsweise sogar bis zu 24 Stunden, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschenderweise gefunden, und dann liegt die Lösung all dieser Aufgaben, daß kosmetische Desodorantien, enthaltend ein synergistisch wirksames Gemisch aus
A) mindestens einem mehrwertigen Alkohol mit 5 bis 15 Kohlenstoffatomen und
B) mindestens einem Citronensäuretrialkylester
die Nachteile des Standes der Technik nicht aufweisen.

Überraschend zeigen die erfindungsgemäßen kosmetischen Desodorantien bei den durchgeführten 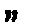 Sniffingtests" auch nach 24 Stunden noch eine ausgezeichnete geruchsreduzierende Wirkung, ohne daß nachteilige Wirkungen wie Hautirritationen oder ähnliches festgestellt werden.

Bevorzugt ist der mehrwertige Alkohol der Komponente (A) ein Diol, wobei 1,2-Diole besonders vorteilhaft sind. Der mehrwertige Alkohol besitzt vorzugsweise 5 bis 8 Kohlenstoffatome. Vorteilhaft ist der mehrwertige Alkohol ausgewählt aus den Diolen 1,2-Pentandiol, 1,3-Pentandiol, 1,5-Pentandiol, 1,2-Hexandiol, 1,3-Hexandiol, 2,5-Hexandiol, 1,5-Hexandiol, 1,6-Hexandiol, 1,2-Heptandiol, 1,3-Heptandiol, 1,2-Octandiol und 1,3-Octandiol, wobei das 1,2-Pentandiol besonders bevorzugt ist.

Der mehrwertige Alkohol (A) ist vorzugsweise in einer Menge von 1 bis 20 Gew.% und besonders vorteilhaft in einer Menge von 2 bis 12 Gew.% enthalten. Der optimale Mengenbereich liegt bei 6 bis 9 Gew.%.

Bevorzugt weisen die Estergruppen des Citronensäuretrialkylester der Komponente (B) 1 bis 16 Kohlenstoffatome, insbesondere 2 bis 4 Kohlenstoffatome, auf. Vorteilhaft ist der Citronensäuretrialkylester ausgewählt aus Citronensäuretriethylester, Citronensäuretributylester, Citronensäuretricaprylester, Citronensäuretri-C₁₂₋₁₃ -alkylester und Citronensäuretri-C₁₄₋₁₅-alkylester, wobei der Citronensäuretriethylester besonders bevorzugt ist.

Der Citronensäuretrialkylester ist vorzugsweise in einer Menge von 0,01 bis 10 Gew.% und besonders vorteilhaft in einer Menge von 0,1 bis 5 Gew.% enthalten. Der optimale Mengenbereich liegt bei 2 bis 4 Gew.%.

Die erfindungsgemäßen kosmetischen Desodorantien können in Form von Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen aus Deo Roll-on-Flaschen oder in Form von Deo-Stiften (Deo-Sticks) mit fester Konsistenz aus Stifthülsen oder in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Als übliche kosmetische Trägerstoffe zur Herstellung der erfindungsgemäßen desodorierenden Zubereitungen können, neben den Lösungsmitteln Wasser, C₁ bis C₃-Alkoholen, wie z. B. Ethanol, Propanol, Isopropanol, C₂ bis C₃-Polyolen, wie zum Beispiel Ethylenglykol, Propylenglykol, 1,2- oder 1,3-Propandiol und Glycerin; hautpflegende Fett- oder fettähnliche Stoffe, wie Ölsäuredecylester, Polyethylenglykolester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z. B. Polyvinylpyrrolidon, Bentonit, Fettsäuren, Stärke, Polyacrylsäure und deren Derivate, Cellulosederivate z. B. Hydroxyethyl- oder Hydroxypropylcellulose, Alginate, Vaseline, Paraffinöle; daneben aber auch in kleinen Mengen cyclische Silikonöle wie z. B. Polydimethylsiloxane niedriger Viskosität.

Selbstverständlich kann die erfindungsgemäße desodorierende kosmetische Zubereitung auch Netzmittel oder Emulgatoren aus den Klassen der anionischen, kationischen, amphoteren oder nichtionogenen oberflächenaktiven Substanzen, beispielsweise Fettalkoholsulfate, Fettalkoholethersulfate, Alkylsulfonate, Alkylbenzolsulfate, quaternäre Ammoniumsalze, Alkylbetaine, oxethylierte Alkylphenole, Fettsäurealkanolamide oder oxethylierte Fettsäureester, ferner Trübungsmittel; Zucker wie z. B. D-Glucose, Lösungsvermittler, Stabilisatoren, Puffersubstanzen, Parfümöle, Farbstoffe sowie hautpflegende Bestandteile, wie z. B. kationische Polymere, Lanolinderivate, Cholesterin, Pantothensäure und Betain, enthalten.

Die erwähnten Bestandteile werden in dem Mittel in den für solche Zwecke üblichen Mengen verwendet, z. B. die Netzmittel und Emulgatoren in Konzentrationen von insgesamt 0,1 bis 10 Gew.%, vorzugsweise 0,5 bis 5 Gew.%, die Trübungsmittel, Parfümöle und Farbstoffe in einer Menge von jeweils 0,01 bis 2 Gew.-%, die Puffersubstanzen in einer Menge von insgesamt 0,01 bis 10 Gew.-%, vorzugsweise 0,1 bis 2 Gew.%, die Zucker und Stabilisatoren in einer Menge von jeweils 0,1 bis 8 Gew.-%, während die Verdickungsmittet, Lösungsvermittler und die pflegenden Bestandteile zweckmäßig in einer Menge von insgesamt 0,5 bis 20 Gew.-% in diesem Mittel enthalten sein können.

Die Einsatzmenge der Lösungsmittel in den Zubereitungen beträgt bis zu 98 Gew.%, bevorzugt 50 bis 90 Gew.% und ganz besonders bevorzugt 75 bis 85 Gew.%

Als Treibmittel für erfindungsgemäße, aus Aerosolbehältern versprühbare kosmetische Desodorantien sind die bekannten leichtflüchtigen, verflüssigten Treibmittel, besonders die Kohlenwasserstoffe (Propan, Butan, Isobutan) geeignet, die allein oder in Mischung miteinander eingesetzt werden können. Auch Druckluft, N₂O oder CO₂ ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Zubereitungen in geringer Menge, z.B. 2 bis 5 Gew.%, bezogen auf die Gesamtzusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den erfindungsgemäßen desodorierenden kosmetischen Zubereitungen Antioxidantien (z. B. Ascorbinsäure oder alpha-Tocopherol und seine Derivate) in Mengen von 0,01 bis 0,03 Gew.%, bezogen auf die Gesamtzusammensetzung beigemischt werden.

Es ist von Vorteil, den pH-Wert der erfindungsgemäßen Zubereitungen im sauren bis neutralen Bereich zu wählen. Besonders vorteilhaft wird der pH-Wert der erfindungsgemäßen Zubereitungen im Bereich von 4,5 bis 6,5, insbesondere bei etwa 5,5, gewählt.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Zubereitungen erfolgt in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen, hergestellt und dann emulgiert. Ansonsten sind die üblichen Maßregeln für das Zusammenstellen von kosmetischen Formulierungen zu beachten, die dem Fachmann geläufig sind.

Sollen die erfindungsgemäßen Zusammensetzungen in Pudersprays eingearbeitet werden, so können die Suspensionsgrundlagen dafür vorteilhaft gewählt werden aus der Gruppe Kieselsäuregele (z.B. solche die unter dem Handelsnamen Aerosil erhältlich sind). Kieselgur, Talkum, modifizierte Stärke, Titandioxid, Seidenpulver, Nylonpulver, Polyethenpulver und verwandten Stoffen.

### Beispiel 1

| **Pumpdeo ohne Alkohol** | |
|---|---|
| 8,0 g | 1,2-Pentandiol |
| 3,5 g | Citronensäuretrietylester (Triethylcitrat) |
| 7,0 g | Polyoxyethylen-(9)-polyoxypropylen-(2)-C₁₆₋₁₈-alkylether |
| 1,5 g | Parfümöl |
| 0,5 g | Betainhydrat |
| ad 100,0 g | Wasser |

Dieses Pumpdeo zeigt beim 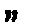 Sniffingtests" auch nach 24 Stunden noch eine ausgezeichnete geruchsreduzierende Wirkung, ohne daß nachteilige Wirkungen wie Hautirritationen oder ähnliches festgestellt werden.

### Beispiele 2 bis 5

| **Pumpsprays mit Alkohol** | 2 | 3 | 4 | 5 |
|---|---|---|---|---|
| 1,2-Pentandiol | - | 5,80 g | 6,00 g | - |
| 1,2-Hexandiol | 4,00 g | - | - | 7,00 g |
| Citronensäuretrietylester | 2,50 g | 3,00 g | - | - |
| Citronensäuretributylester | - | - | 4,00 g | 3,00 g |
| Propylenglykol | 3,00 g | 3,00 g | 3,00 g | 3,00 g |
| PEG-40-Hydriertes Rizinußöl | - | 2,50 g | 2,25 g | - |
| Ethanol | 69,00 g | 65,00 g | 63,00 g | 65,00 g |
| Cetylalkohol-polyethylenglykol-(25)-ether | 3,00 g | - | 2,00 g | 1,00 g |
| Parfümöl | 0,10 g | 0,20 g | 0,20 g | 0,20 g |
| NaOH ad pH = 5,5 | | | | |
| Wasser, demineralisiert | ad 100,00 g | ad 100,00 g | ad 100,00 g | ad 100,00 g |

### Beispiele 6 und 7

| **Roll-on Gele** | 6 | 7 |
|---|---|---|
| 1,2-Pentandiol | - | 5,80 g |
| 1,2-Hexandiol | 4,00 g | - |
| Citronensäuretrietylester | 2,50 g | |
| Citronensäuretributylester | - | 3,00 g |
| Dipropylenglykol | 4,00 g | 5,00 g |
| PEG-40-hydriertes Rizinußöl | 2,50 g | 2,50 g |
| Ethanol | 50,00 g | 55,00 g |
| Hydroxyethylcellulose | 0,50 g | 0,50 g |
| Parfümöl | 0,10 g | 0,20 g |
| NaOH ad pH = 5,7 | | |
| Wasser, demineralisiert | ad 100,00 g | ad 100,00 g |

### Beispiele 8 und 9

| **Wachsstifte** | 8 | 9 |
|---|---|---|
| 1,2-Pentandiol | - | 10,00 g |
| 1,2-Hexandiol | 5,00 g | - |
| Citronensäuretrietylester | - | 5,00 g |
| Citronensäuretributylester | 3,00 g | - |
| Trilaurin | 37,50 g | 37,50 g |
| Glycerylstearat, selbstemulgierend | 9,00 g | 10,00 g |
| Bienenwachs | 22,00 g | 23,00 g |
| Parfümöl | 0,10 g | 0,20 g |
| Caprylic/Capric-Triglycerid | ad 100,00 g | ad 100,00 g |

Die Bestandteile werden bei ca. 75°C aufgeschmolzen, gut vermischt und in geeignete Formen gegossen.

## Patentansprüche

1. Kosmetisches Desodorans, enthaltend ein Gemisch aus
A) mindestens einem mehrwertigen Alkohol mit 5 bis 15 Kohlenstoffatomen und
B) mindestens einem Citronensäuretrialkylester.

2. Mittel nach Anspruch 1, dadurch gekennzeichnet, daß der mehrwertige Alkohol der Komponente (A) ein Diol ist.

3. Mittel nach einem der Ansprüche 1 oder 2, dadurch gekennzeichnet, daß der mehrwertige Alkohol 5 bis 8 Kohlenstoffatome besitzt.

4. Mittel nach einem der Ansprüche 1 bis 3, dadurch gekennzeichnet, daß mehrwertige Alkohol ein 1,2-Diol ist.

5. Mittel nach einem der Ansprüche 1 bis 3, dadurch gekennzeichnet, daß mehrwertige Alkohol ausgewählt ist aus den Diolen 1,2-Pentandiol, 1,3-Pentandiol, 1,5-Pentandiol, 1,2-Hexandiol, 1,3-Hexandiol, 2,5-Hexandiol, 1,5-Hexandiol, 1,6-Hexandiol, 1,2-Heptandiol, 1,3-Heptandiol, 1,2-Octandiol und 1,3-Octandiol.

6. Mittel nach Anspruch 5, dadurch gekennzeichnet, daß das Diol das 1,2-Pentandiol ist.

7. Mittel nach einem der Ansprüche 1 bis 6, dadurch gekennzeichnet, daß der mehrwertige Alkohol in einer Menge von 1 bis 20 Gew.% enthalten ist.

8. Mittel nach einem der Ansprüche 1 bis 7, dadurch gekennzeichnet, daß der Citronensäuretrialkylester der Komponente (B) an den Estergruppen jeweils 1 bis 16 Kohlenstoffatome aufweist.

9. Mittel nach einem der Ansprüche 1 bis 8 dadurch gekennzeichnet, daß der Citronensäuretrialkylester ausgewählt ist aus Citronensäuretriethylester, Citronensäuretributylester, Citronensäuretricaprylester, Citronensäure-tri-C₁₂₋₁₃-alkylester und Citronensäure-tri-C₁₄₋₁₅-alkylester.

10. Mittel nach einem der Ansprüche 1 bis 9, dadurch gekennzeichnet, daß der Citronensäuretrialkylester in einer Menge von 0,01 bis 10 Gew.% enthalten ist.

11. Verwendung eines Gemisches aus
A) mindestens einem mehrwertigen Alkohol mit 5 bis 15 Kohlenstoffatomen und
B) mindestens einem Citronensäuretrialkylester
als desodorierend wirkendes Prinzip für kosmetische Desodorantien.

12. Verfahren zur Bekämpfung des durch mikrobielle Zersetzung apokrinen Schweißes hervorgerufenen menschlichen Körpergeruches, dadurch gekennzeichnet, daß eine wirksame Menge an einem Gemisch aus (A) mindestens einem mehrwertigen Alkohol mit 5 bis 15 Kohlenstoffatomen und (B) mindestens einem Citronensäuretrialkylester, gegebenenfalls in einem geeigneten kosmetischen Träger, auf die Haut aufgetragen wird.
